(19) Europäisches Patentamt
European Patent Office
Office européen des brevets

(11) **EP 4 025 286 B1**

(12) **EUROPÄISCHE PATENTSCHRIFT**

(45) Veröffentlichungstag und Bekanntmachung des Hinweises auf die Patenterteilung:
**23.04.2025 Patentblatt 2025/17**

(21) Anmeldenummer: **20786319.2**

(22) Anmeldetag: **04.09.2020**

(51) Internationale Patentklassifikation (IPC):
*A61M 25/00* *(2006.01)*    *A61M 25/01* *(2006.01)*
*A61M 25/04* *(2006.01)*    *A61F 2/04* *(2013.01)*
*A61M 27/00* *(2006.01)*    *A61B 46/20* *(2016.01)*
*A61B 46/23* *(2016.01)*    *A61B 46/00* *(2016.01)*

(52) Gemeinsame Patentklassifikation (CPC):
**A61M 25/04; A61B 46/20; A61B 46/23;**
**A61B 46/30; A61M 25/0017; A61M 25/0102;**
A61B 46/10; A61M 25/0041; A61M 27/00

(86) Internationale Anmeldenummer:
**PCT/IB2020/058241**

(87) Internationale Veröffentlichungsnummer:
**WO 2021/044354 (11.03.2021 Gazette 2021/10)**

(54) **VORRICHTUNG FÜR DIE URETHRALE APPLIKATION EINER LUBRIFIZIERENDEN BZW. LUMENAUFRICHTENDEN SUBSTANZ DURCH EINE RETRAHIERBARE EINFÜHRHILFE**

DEVICE FOR URETHRAL APPLICATION OF A LUBRICATING AND/OR LUMEN-STRAIGHTENING SUBSTANCE THROUGH A RETRACTABLE INSERTION AID

DISPOSITIF D'ADMINISTRATION, PAR VOIE URÉTRALE, D'UNE SUBSTANCE LUBRIFIANTE OU INDUISANT LE DÉPLOIEMENT D'UNE LUMIÈRE, PAR L'INTERMÉDIAIRE D'UN ACCESSOIRE D'INTRODUCTION RÉTRACTABLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.09.2019 DE 102019006229**

(43) Veröffentlichungstag der Anmeldung:
**13.07.2022 Patentblatt 2022/28**

(73) Patentinhaber: **Advanced Medical Balloons GmbH**
**68753 Waghäusel (DE)**

(72) Erfinder: **GÖBEL, Fred**
**67346 Speyer (DE)**

(74) Vertreter: **Küchler, Stefan**
**Stefan T. Küchler**
**Patentanwalt**
**Färberstraße 20**
**90402 Nürnberg (DE)**

(56) Entgegenhaltungen:
EP-A2- 0 935 977    SE-A1- 1 650 363
US-A- 4 155 364    US-A- 4 571 239
US-A- 5 441 485    US-A1- 2012 184 942
US-A1- 2017 238 951    US-A1- 2018 071 486

## Beschreibung

Die Erfindung richtet sich auf eine Vorrichtung zum Ableiten von Urin aus der Blase eines Patienten,

Blasenkatheter zur kontinuierlichen, geschlossenen Ableitung von Urin aus der Blase eines Patienten in ein Sammelgefäß sind in der medizinischen Praxis etabliert und in verschiedenen Ausführungsformen bekannt, Blasenverweilkatheter werden in der Regel durch trans-urethrale Katheterisierung in die Harnblase eingebracht. Im Gegensatz zur Katheterisierung der Blase mit Einmalkathetern, bei der intermittierend, also nur für die tatsächliche Dauer der Entleerung der Blase, ein katheterartiger Schlauch durch die Harnröhre in das Innere der Blase vorgeschoben wird, sind Katheter zur dauerhaften trans-urethralen Ableitung von Urin an ihrem distalen Ende typischerweise mit einem ballonartigen Ankerelement (Retentionsballon) ausgestattet, dessen primäre Funktion die Sicherung der Position des den Urin aufnehmenden Katheterendes innerhalb der Blase ist.

Bei der überwiegenden Mehrzahl der bekannten Bautypen sogenannter Dauerkatheter bestehen diese Retentionsballons aus elastisch dehnbaren Schlauchmaterialien, die dem Schaftkörper des Katheters eng angeschmiegt und möglichst ohne jede Präformation, als glatte, faltungsfreie, zylindrisch geformte Schlauchkörper (Sleeves) aufsitzen. Durch Befüllung mit einem nicht-kompressiblen, flüssigen Medium dehnt sich aus dem zylindrischen Schlauchkörper bei entsprechend hohem Befülldruck ein sich annähernd sphärisch ausformender Ballonkörper auf. Nachteilig bei derartigen, durch hohe Fülldrucke aufgedehnten Ankerballonen ist vor allem die durch die Dehnung der Hülle resultierende, harte Konsistenz des Ballons. Bei längerer Liegedauer sind im Auflagebereich solcher rigiden, "harten" Ballons auf dem Blasenboden, insbesondere im Bereich des sogenannten Blasen-Trigonums, erosive Veränderungen und zum Teil auch ausgeprägte Ulzerationen der Blasenschleimhaut die Folge. Bei anhaltender Präsenz von Bakterien im Urin können sich auf der Grundlage solcher geweblichen Defekte chronische Entzündungen des Harntraktes entwickeln. Die aktuelle Forschung auf dem Gebiet der "urinary tract infections" (UTI) wertet ein möglichst intaktes, möglichst nicht erodiertes Epithel der Blase und auch der Harnröhre (Urethra) als eine wesentliche Voraussetzung für die Vermeidung chronischer Harnwegsinfekte.

Neben herkömmlichen, sleeve-basierten Kathetern sind in der Literatur auch Retentionselemente auf der Basis vorgeformter Ballonkomponenten bekannt. Derartige Komponenten werden beispielsweise in EP1448259 oder PCT/IB2015/002282 vorgestellt. Die Retention wird hier durch bereits bei der Herstellung des Katheters vollständig oder auch partiell auf das für die jeweilige Funktion erforderliche Arbeitsmaß ausgeformte Balionkomponenten gewährleistet. Die Ballonelemente bestehen aus membranartigen dünnwandigen, aber hoch-festen Materialien, welche im nichtbefüllten Zustand derart gering auf dem Katheterschaft auftragen, dass die ausgeformte Ballonkomponente beim Einführen des Katheters komfortabel und ohne Irritation durch die Harnröhre vorgeschoben werden kann.

Die Präambel des Anspruchs 1 wird durch EP0935977A2 offenbart.

In der jüngeren Literatur zum Thema UTI wird zudem vielfach auf die traumatisierende Wirkung herkömmlicher Katheterspitzen auf das der Spitze exponierte Blasendach hingewiesen. Die in der Regel relativ starre, über den Retentionsballon hinaus, fingerartig in das Blasenlumen reichende Spitze gräbt sich bei anhaltender Exposition in das Gewebe des Blasendachs ein und führt so regelhaft zu weiteren, unmittelbar katheterassoziierten Läsionen,

Es sind vielfältig gestaltete Ausführungsformen von Katheterspitzen bekannt, die insbesondere beim Einführen bzw. Vorschieben des Katheters durch die Harnröhre Läsionen der Harnröhrenwandung verhindern und die Katheteranlage insgesamt erleichtern sollen. Einige Bautypen versuchen zudem Verletzungen des Blasendaches vorzubeugen. Beispielsweise werden Doppelballon-Anordnungen beschrieben, wie beim Typ Duette der Firma Poiesis Medical Ltd., wobei die Spitze des Katheters in schützender Weise von einem sphärischen, ballonartigen Element aufgenommen wird, während ein weiteres Ballonelement retinierend dem Blasenboden aufliegt. Die Ableitung des Urins erfolgt bei Duette durch eine Öffnung des Katheterschaftes zwischen den beiden Ballons,

Eine weitere, den Harntrakt direkt traumatisierende Wirkung liegt im Aufbau des urinableitenden Schaftsegmentes konventioneller Katheters begründet. Der Schaft schließt sich nach proximal an den in der Blase retinierenden Ballon an und leitet den Urin trans-urethral ab, Die Schaftelemente bestehen typisch aus relativ dickwandig ausgeführtem Schlauchmaterial auf Silikonbasis oder aus Naturkautschuk basierten Materialien. Die dickwandige Ausführung der Schaftwandung gewährleistet zum einen, dass sich das urinableitende Lumen bei axialer Biegung oder Verwindung des Schaftes nicht durch Knickung verschließt. Zum anderen stellt der dickwandige Schaftaufbau sicher, dass es bei der elastischen Aufdehnung des Ballon-Sleeves zum sphärischen Ballon, bei den im Ballon wirkenden, hohen Drucken nicht zum Kollaps des urinableiten Lumens des Schaftes kommt. Insbesondere die durch den Ballonfülldruck erforderlichen Schaftwandstärken schränken die baulich erreichbaren Durchmesser des urinableitenden Lumens ein. Kleinlumige Ableitungen neigen dazu durch fortwährende Ablagerung von Urinbestandteilen frühzeitig zu verkrusten bzw. sich sukzessive zu verschließen. Um möglichst lange Anwendungsperioden zu erreichen, strebt man daher möglichst große Innendurchmesser an, die wiederum große Schaftaußendurchmesser erfordern, Die bei herkömmlichem Katheterdesign somit erforderliche Näherung des Schaftaußendurchmessers an den Durchmesser der Harnröhre führt im Verbund mit der Steifigkeit des Schaftmaterials häufig zu ausgeprägten Erosionen des die Harnröhre auskleidenden Epithels, wodurch bei langer Liegedauer regelhaft entzündliche Prozesse, und in der Folge narbige Strikturen der Harnröhre resultieren.

Ein weiterer, die Entstehung chronischer Harnwegsentzündungen unterstützender Faktor ist die Ansammlung von sogenanntem Blasenrestharn. Restharn entleert sich aus der Blase nur verzögert und neigt daher zur Akkumulation größerer Bakterienmengen. Es sind verschiedene Ausführungsformen bekannt, die das "pooling" von Harn vermindern, überwiegend durch die Ableitung des Urins über Öffnungen des Katheterschaftes, welche besonders nahe zum Blasenboden hin positioniert sind.

Um Verletzungen bei der Katheteranlage zu vermeiden, werden mit gutem Erfolg gelartige Substanzen verwendet, Sie reduzieren zum einen die Friktion zwischen der Katheterschaftoberfläche und dem Epithel der Harnröhre, Über diesen lubrifizierenden Effekt hinaus, ermöglichen Gele eine passagere Aufrichtung bzw. Füllung des Harnröhrenlumens. Im mehr oder weniger zirkulär aufgerichteten Zustand des Harnröhrenlumens können die Katheterspitze und der Katheterschaft somit optimal reibungsarm durch das Lumen der Harnröhre eingeführt werden. Blasenkatheter werden häufig im Set, mit allen für die Katheteranlage erforderlichen Utensilien verpackt. Die anwendungsfertigen Sets sind in der Regel unnötig umfangreich, in vielen Fällen in der Handhabung nicht mehr selbsterklärend und behindert die Anwendung des Produktes.

Die Aufgabe der Erfindung ist die Vermeidung der vorangehend beschriebenen Nachteile des Standes der Technik.

Zur Lösung dieses Problems sieht die Erfindung im Rahmen einer Vorrichtung zum Ableiten von Urin aus der Blase eines Patienten eine Funktionseinheit vor, umfassend

a) ein schaftförmiges, in der Blase und in der Harnröhre nach Art eines Katheters platzierbares, drainierendes Verweilteil mit wenigstens einer Drainageöffnung im Bereich der distalen Spitze des schaftförmigen Verweilteils; sowie

b) eine in das schaftförmige Verweilteil eingesteckte, retrahierbare, die urethrale Einführung und Passage erleichternde Einführhilfe von rohr- oder schlauchartigem Aufbau mit einer apikalen Öffnung zur endständigen Freisetzung einer lubrifizierenden und/oder lumen aufrichtenden Substanz durch die Öffnung.

Im Rahmen der vorliegenden Patentanmeldung werden die Begriffe "Verweilteil", "Verweileinheit", "Katheter" und "Blasenkatheter" weitgehend synonym verwendet,

Die vorliegende Erfindung ist durch Anspruch 1 definiert und beschreibt insbesondere die anwendungsoptimierende Verbindung eines Blasenkatheters mit einem, in das urinableitende Lumen des Katheters eingeschobenen, reversibel entnehmbaren Einführelement, welches den Katheterschaft für die urethrale Einführung stabilisiert und die handhabungsfreundliche intra-uretihrale Einbringung von lubrifizierendem bzw. lumenaufrichtendem Gel gestattet. Der einfachen Handhabung des Katheters wird im Rahmen der Erfindung durch entsprechende bauliche Modifikationen und akzessorisch Ergänzungen Rechnung getragen. Insbesondere bietet die Erfindung die Möglichkeit einer mit einer einzigen Hand geführten Katheterisierung, die im optimalen Falle die Lubrifizierung der Katheterspitze, das Einführen des vorderen Katheterdrittels, die Lumenaufrichtung der Harnröhre durch Gelapplikation, das sukzessive Vorschieben des Katheters durch ein zangenartiges Greifelement, sowie das Blocken des Retentionsballons umfasst. Die besondere Ausführung der erfindungsgemäßen Vorrichtung gestattet es den Umfang eines Kathetersets auf ein Mindestmaß zu reduzieren.

Die Erfindung beschreibt ferner die besonders atraumatische Gestaltung sämtlicher intrakorporal platzierten Komponenten eines Blasenkatheters. Insbesondere soll sich der Katheterschaft möglichst gewebefreundlich, und Erosionen vermeidend in der Harnröhre verhalten, Perforierende Verletzungen des Blasendachs durch die Spitze des Katheters sollen vermieden werden. Der Katheterballon soll einen möglichst gut retinierenden und schmiegend dichtenden Effekt im Bereich des Blasenausgangs haben, wobei eine erodierende Wirkung der Ballonwandung auf das Epithel möglichst gering ist. Das den Urin ableitende Lumen des Katheterschaftes soll optimal groß sein. Ferner soll das pooling von Restharn in der Blase minimiert werden.

Die Erfindung beschreibt insbesondere eine Kombination eines dünnwandig bzw. großlumig ausgeführten, effizient urinableitenden, atraumatisch in der Harnröhre verweilenden Schaftschlauchs, mit einer in den dünnwandigen Schaftschlauch eingeschobenen Einführhilfe, die neben der für die Katheteranlage erforderlichen, stabilisierenden Versteifung des Katheterschaftes, die spitzennahe Freisetzung einer lubrifizierenden und/oder lumenaufrichtenden Substanz in die Harnröhre ermöglicht. Die Einführhilfe wird nach der Sicherung des Katheters durch Befüllung des Katheterballons aus dem Katheterlumen herausgezogen, so dass in der Harnröhre ein möglichst geschmeidiger, reibungsminimierter Schlauchkörper zu liegen kommt, der die typischen, erosionsbedingten Läsionen der Urethra bei konventioneller Katheterisierung vermeidet.

Die Einführhilfe weist in der bevorzugten Bauform, als schlauch- oder rohrartiger Körper, ein ca. 1,0 bis 3,0 mm messendes Innenlumen auf, durch das eine lubrifizierende und/oder lumenaufrichtende Substanz im Bereich der Spitze des die Einführhilfe aufnehmenden Blasenkatheterschaftes freigesetzt wird. Die jeweilige Substanz tritt dabei möglichst nahe zur urinableitenden Öffnung des Blasenkatheters beabstandet in das drainierende Lumen des Katheters aus.

Alternativ kann die Spitze der Einführhilfe in axialer Verlängerung des in der Blase verweilenden Katheteranteils, über eine sich nach frontal öffnende Mündung des Katheterschaftes, hinausreichen. Die Einführhilfe kann bei einer derartigen Ausführung im Bereich ihrer Spitze mit einer der vielfältigen, in der trans-urethralen Katheterisierung üblichen Spitzenausformungen versehen sein, die die Passage des Katheters durch kritische Biegungen im Harnröhrenverlauf erleichtern. Auch hier weist das Einführelement ein Innenlumen zur Applikation einer lubrifizierenden und oder lumenaufrichtenden Substanz auf, wobei die Abgabe in diesem Falle direkt aus einer Öffnung des Einführelementes in die Harnröhre erfolgt. Im Anschluss an die Sicherung des Katheters in der Blase wird die Einführhilfe entfernt. Nach Retraktion der Einführhilfe stellt sich im bevorzugten Falle im vorderen Ballonradius des Katheterballons eine trichterartige Mündung ein, wobei sich sie Öffnung des Katheterschaftes bei der Befüllung des Ballons spontan am Boden des Trichters positioniert.

Alternativ kann der Katheterschaft eine kurze, stutzenartige Spitzenformation aufweisen, die jedoch derart gestaltet ist, dass sie im in situ platzierten Ballon den vorderen Radius des Ballons nicht überragt.

Die Erfindung beschreibt ferner eine besondere Ausformung der Katheterspitze, die eine wellschlauchartig, korrugiert ausgeformte Wandung aufweist, die die Spitze frei rotierbar bzw. in jede Richtung verbiegbar macht, und somit zum einen die Passage der Harnröhre vereinfacht, und zum anderen innerhalb der Blase das allseitige axiale Umbiegen der Spitze gestattet, so dass das Blasendach beim Aufliegen auf dem distalen Katheterende nicht von einer spitz auf sie zu weisenden Formation irritiert und lädiert wird.

Der Ballonkörper ist bei allen Ausführungsformen der erfindungsgemäßen Vorrichtung zur Reduktion von Restharnbildung bevorzugt möglichst flach bzw. diskoid ausgeformt.

Im Sinne einer optimal vereinfachten Handhabung bei der Katheterisierung kann die lubrifizierende und/oder lumenaufrichtende Substanz im ersten Handhabungsschritt in eine haubenartige bzw. tütenartige Hülle eingespritzt werden, die dem distalen Katheterende bzw. dem distalen Ende der Einführhilfe mit einem schmalen, zwischenliegenden Spaltraum aufsitzt und die Substanz möglichst gleichförmig in diesem Spaltraum um die Katheterspitze bzw. das distale Katheterende herum verteilt. Die Hülle wird dann, unmittelbar vor der Einführung des Katheters in die Harnröhre, nach frontal hin abgezogen.

Der durch die Einführhilfe stabilisierend versteifte Katheter wird etwa 8 bis 10 cm in die männliche Harnröhre in quasi lanzierend geführter Weise eingeführt, Um die weitere Passage des Katheters in optimal atraumatischer Weise zu ermöglichen, wird im Anschluss an die Einführung des vorderen Katheterabschnittes eine zweite Gel-Menge in die Harnröhre injiziert, die das Lumen des Organs aufrichtet und so Verletzungen insbesondere dort vermeidet, wo die männliche Harnröhre einen besonders kurvenartig gewundenen Verlauf nimmt.

Dem Verschluss des Drainagelumens durch Ablagerungen von Harnbestandteilen wird im Rahmen der Erfindung durch eine maximal großlumige Ausführung des urinableitenden Lumens begegnet, Hierzu wird die Wandung des trans-urethralen Segmentes des Katheters so weit wie möglich, im extremen Fall auf Folienstärke reduziert. Zur Vermeidung lumenverschließender Knickungen oder Verwindungen eines derartig dünnwandigen Schaftes kann dieser über die gesamte Länge hinweg, oder auch nur in bestimmten, mechanisch relevanten Abschnitten, mit einem lumenstabilisierenden, wellschlauchartigen Profil versehen sein. Im Verbund mit Materialien mit hoher elastischer Rückstellkraft, wie z.B. Polyurethan kann ein derartig profilierter Schaft mit spontan lumenaufrichtenden Eigenschaften ausgestattet werden, die zwar eine elastisch wirkende, geschmeidige Verbiegung des Schaftes, sowie einen leicht herbeizuführenden, passageren, radialen Kollaps des Lumens ermöglichen, andererseits aber eine zuverlässige, spontan lumenaufrichtende und entwindende Wirkung sicherstellen.

Zusätzlich sind in Kombination mit schlauchfolienartig dünnwandigen Schaftwandungen beispielsweise auch stabartige, schlauchartige, rohrgeflechtartige Strukturen vorstellbar, die in torsionshemmender, stabilisierender Weise, frei im Inneren eines derartigen Drainageschlauches verlaufen.

Die Erfindung verwendet bevorzugt speziell ausgeformte Ballonkomponenten, die beispielweise in PCT/IB2015/002282 beschrieben werden. Diese, aus mikrodünnwandiger, aber gleichzeitig hoch formstabiler Weichfolie hergestellten Ballons werden vorzugsweise unvollständig, nur schlaff mit Luft befüllt. Bedingt durch die Platzierung im schlaffen, nicht-gedehnten Zustand können traumatisch wirkende Abtragungen des die Blase auskleidenden Epithels weitgehend vermieden werden. Bei einer ungedehnten, schlaffen Befüllung des Ballons wird nur die jeweils in der Blase wirkende Kraft vom Ballon aufgenommen. Die Fülldrucke liegen dabei im niedrigen Millibar-Bereich, Sie überschreiten im nicht belasteten Zustand des Ballons den in der Blase des Patienten herrschenden, intravesikalen Druck nicht oder nur gering.

Wie in der PCT/IB2015/002282 beschrieben, kann ein derartiger Ballon auch mit einem urethralen, im Durchmesser verjüngten, zylindrisch geformten Ausläufer versehen sein. Der retinierende, vesikale Ballonanteil geht hier also nach proximal in einen dichtenden, urethralen Ballonanteil über. Der kombinierte, vesikal-urethrale Ballonkörper wird im urethralen Segment vorzugsweise mit einem "residualen" Durchmesser versehen, der den Durchmesser des Harnröhrenlumens um das ca. 0,5 bis 1,5 fache, bevorzugt um das 0,5 bis 1,0 fache überschreitet. Der so im Durchmesser überdimensionierte Ballonanteil legt sich damit der Harnröhrenwandung spannungsfrei, weitgehend größenunabhängig, unter Ausbildung einer radial orientierten Faltung bzw. Einstülpung der Hüllenwandung an. Durch das residuale Übermaß wird sichergestellt, dass alle Harnröhrenanteile in schlaff tamponierender Weise gedichtet werden können. Durch den residualen Durchmesser können zudem Kaliberschwankungen des Harnröhrenlumens ausgeglichen werden.

Als alternative, weniger bevorzugte Ausführung kann der urethrale Anteil des ausgeformten Ballonkörpers derart bemessen werden, dass er den Durchmesser der jeweiligen Harnröhre im Wesentlichen entspricht oder diese unterschreitet.

Die Befüllung der erfindungsgemäß verwendeten Katheterballons erfolgt vorzugsweise mit einem gasförmigen Medium bzw. mit Luft. Die Zuleitung des Füllmediums erfolgt über eine in die Schaftwandung integrierte Zuleitung, kann aber auch durch frei im Schaftlumen verlaufende Zuleitungen erfolgen.

Die Erfindung berücksichtigt weniger bevorzugt auch Bauformen, die auf die beschriebenen, vorgeformten Ballonkomponenten verzichten und stattdessen konventionelle, sich aus einem Schlauchstück elastisch aufspannende Retentionskomponenten aufweisen.

Das Verweilteil und die Einführhilfe können aus Werkstoffen bestehen, welche die gleiche Härte aufweisen, Bevorzugt werden jedoch Ausführungsvarianten, bei denen sich die Materialien des Verweiltteils und der Einführhilfe zumindest durch ihre Härte voneinander unterscheiden.

Da beide Teile aus Kunststoffen bestehen, bevorzugt aus Thermoplasten oder Duroplasten oder Elastomeren, wird die Materialhärte bevorzugt als Shore-Härte gemessen. Besonders bevorzugt werden dabei die Härtewerte Shore A oder Shore D.

Die Shore-Härte y des Materials der Einführhilfe sollte der Shorehärte x des Materials des Verweilteils entsprechen oder jene überschreiten:

$$y = x,$$

oder

$$y > x,$$

oder

$$y \geq x.$$

Die Differenz der Shore-Härten, insbesondere der Härten nach Shore A, sollte folgendermaßen definiert sein:

$$y - x \geq 0,$$

oder:

$$y - x \geq 1,$$

oder:

$$y - x \geq 2,$$

oder:

$$y - x \geq 5,$$

oder:

$$y - x \geq 10,$$

oder:

$$y - x \geq 20.$$

Ein beispielhaftes, nicht beanspruchtes Verfahren zur Katheterisierung der Blase eines Patienten zeichnet sich dadurch aus, dass eine lubrifizierende und/oder lumenaufrichtende Substanz aus einem kompressiblen Reservoirbehälter freigesetzt wird infolge einer digitalen oder manuellen Kompression des Reservoirbehälters.

Dabei wird eine anwendungsfertig vorbereitete Kathetervorrichtung verwendet, die ein schaftförmiges, in der Blase und in der Harnröhre nach Art eines Katheters platzierbares, drainierendes Verweilteil mit einer Drainageöffnung im Bereich der distalen Spitze des Schaftes umfasst sowie eine in das Verweilteil eingesteckte, retrahierbare, die urethrale Einführung und Passage erleichternde Einführhilfe von rohr- oder schlauchartigem Aufbau mit einer apikalen Öffnung zur endständigen Freisetzung der lubrifizierenden und/oder lumenaufrichtenden Substanz, an deren proximalem Ende der Reservoirbehälter mit der lubrifizierenden und/oder lumenaufrichtenden Substanz angeschlossen oder anschließbar ist.

In den beigefügten Abbildungen werden verschiedene Ausführungsformen der erfindungsgemäßen Blasenkatheter-Vorrichtung, sowie Kombinationen mit besonderen, funktionellen Zubehörkomponenten für die einhändige Durchführung der Katheterisierung beschrieben. Hierbei zeigt:

Abb. 1     eine Ausführungsform, bei der ein substanzapplizierendes bzw. den Katheterschaft stabilisierendes Einführelement in das urinableitende Lumen des Blasenkatheters eingeschoben ist;

Abb. 2     eine Ausführungsform, bei der ein substanzapplizierendes bzw. stabilisierendes Einführelement in das urinableitende Lumen des Blasenkatheters derart eingeschoben wird, dass die Spitze des Einführelementes über die distale, endständige Öffnung des im Körper verweilenden Katheterschaftes hinausreicht;

Abb. 2a    eine besondere, mit einem welligen Profil ausgeformte. Ausführung der Spitze des Einführelementes;

Abb. 26    eine entsprechende, wellig ausgeformte Ausführung des im Körper verweilenden, distalen Katheterendes;

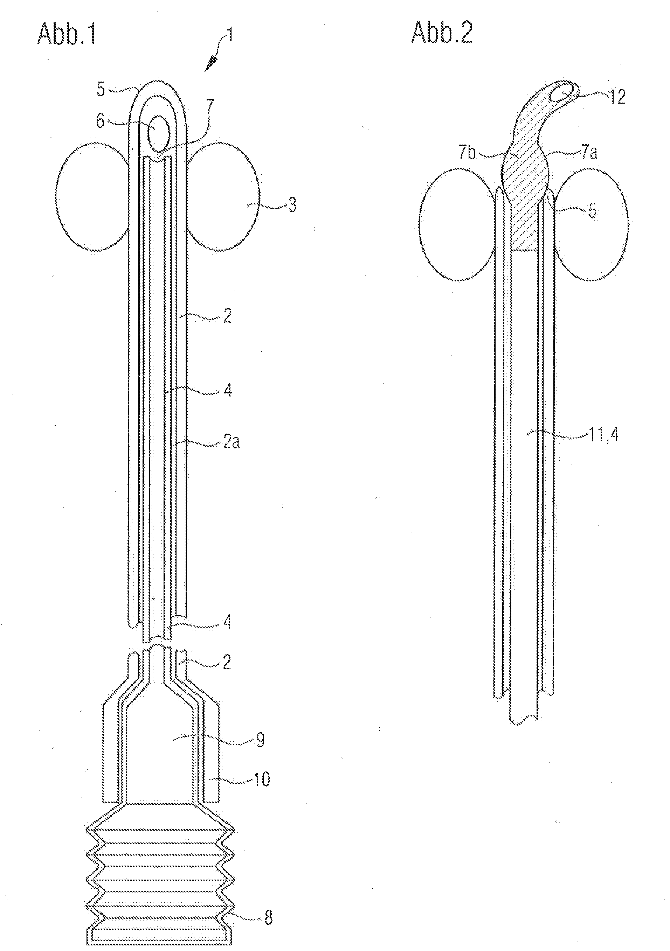

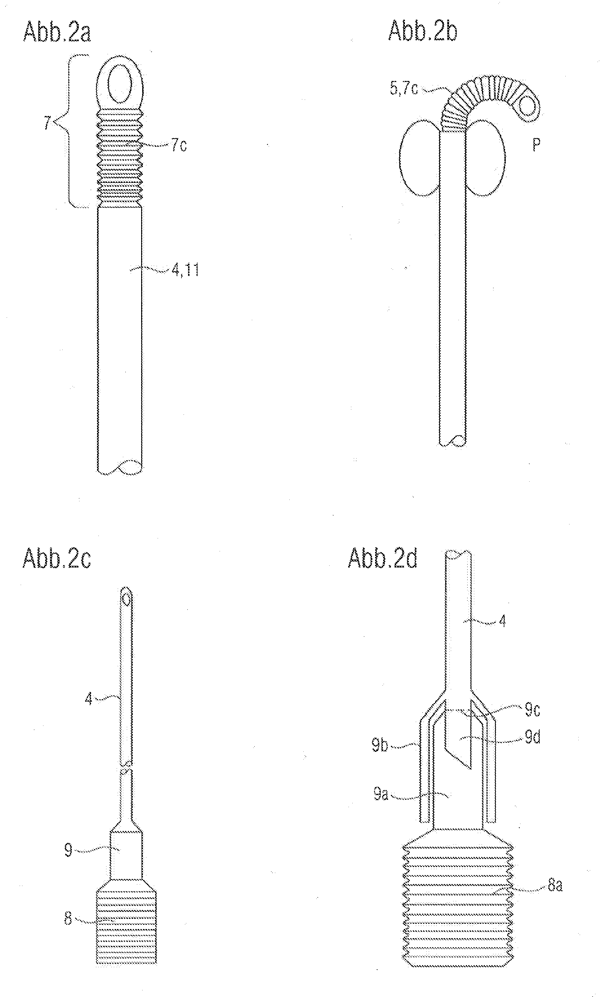

Abb. 2c    ein aus einem einzigen Stück ausgeformtes Einführelement mit integriertem, endständigen, kompressiblen Substanzreservoir;

Abb, 2d    eine besondere Ausführung eines Einführelementes, welches ein endständiges, trichterartiges Aufnahmestück für das Einstecken und perforierende Eröffnen eines baulich separaten Reservoirbehälters aufweist;

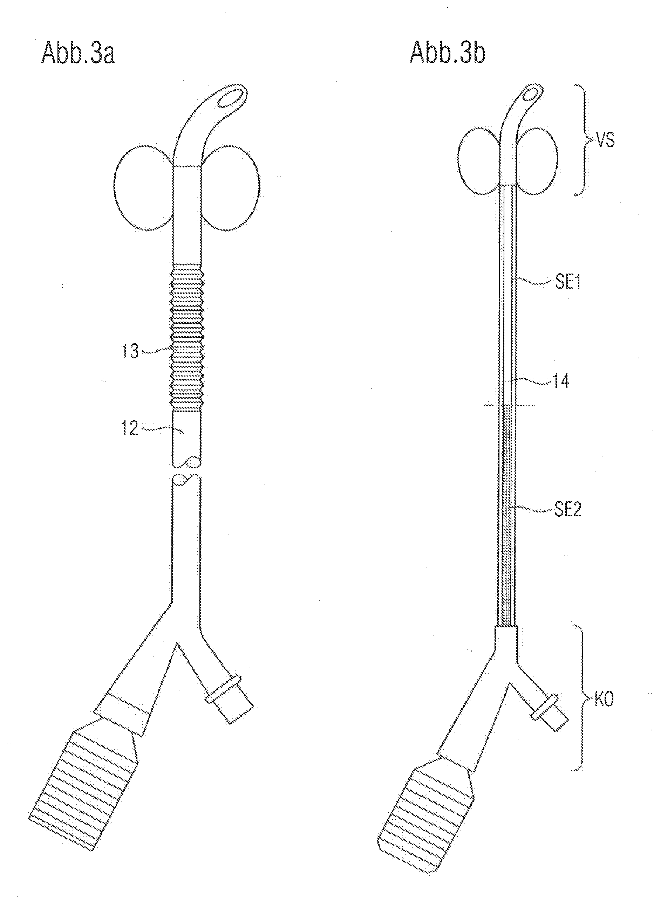

Abb. 3a    eine besondere, in Abschnitten oder über den gesamten Katheterschaft hinweg, wellig ausgeformte Ausführung des Katheterschaftes;

Abb. 3b    eine weitere Ausführungsform des Katheterschaftes, wobei dieser eine besonders dünnwandige, schlauchfolienartige Beschaffenheit aufweist;

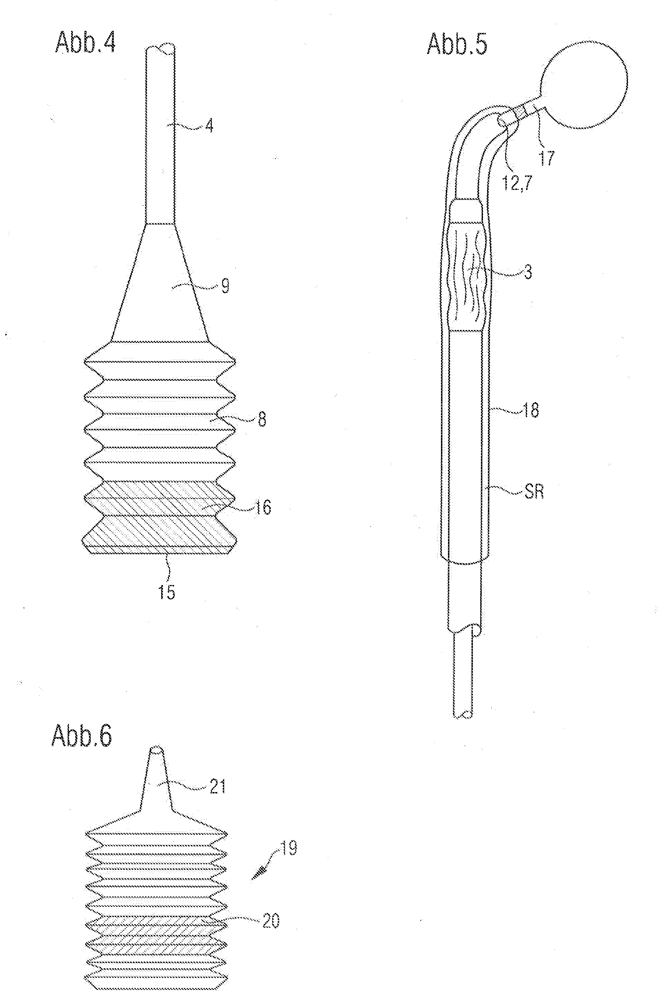

4Abb. 4    einen in rastender Weise stauchbaren Reservoirbehälter für lubrifizierende und/oder lumenaufrichtende Substanzen;

Abb. 5     eine besondere Anordnung eines haubenartigen Elementes für die Lubrifizierung der Katheterspitze;

Abb. 6     eine balgartig stauchbare Befüllvorrichtung zum Befüllen des Katheterballons;

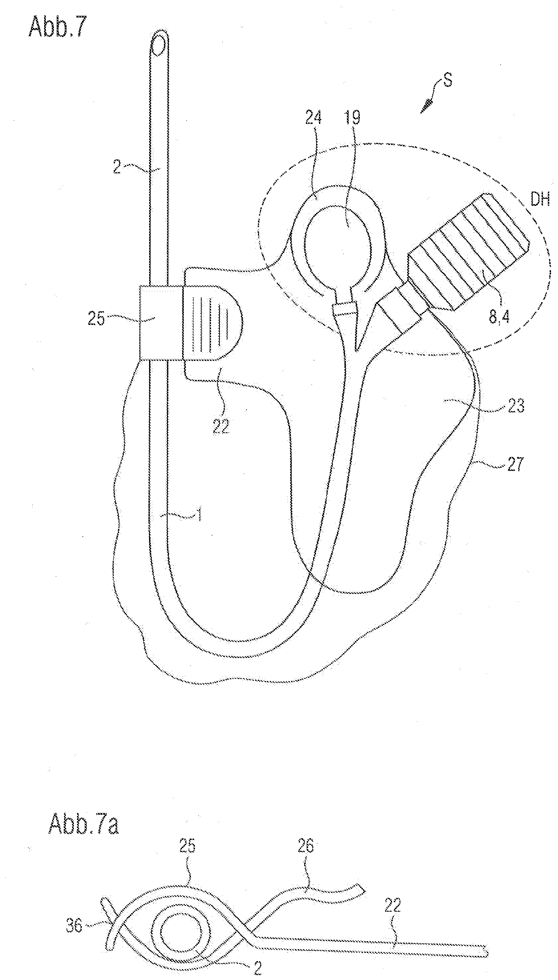

Abb. 7     eine Haltevorrichtung zur u-förmig geschlungenen Aufnahme eines Katheters für die optimierte, anwendungsfertige Einführung des Katheters mit nur einer Hand;

Abb. 7a    einen besonderen Pinzetten- bzw. Zangenmechanismus für das Greifen des Katheterschaftes; sowie

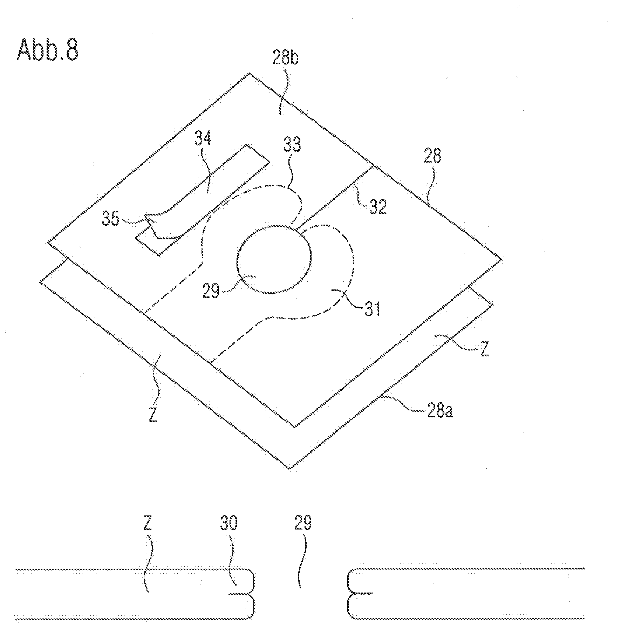

Abb. 8     eine besondere Ausführungsform eines zweilagigen Lochtuches, welches der Anwender handschuhartig aufnehmen und an die zu katheterisierende Öffnung führen kann.

Abb.1 stellt eine Ausführungsvariante der Kathetervorrichtung 1 dar, bei der Katheterschaft 2 der Vorrichtung mit einem, bereits bei der Herstellung auf das für die Retention des Katheters in der Blase erforderlichen Arbeitsmaß ausgeformten Ballon 3 ausgestattet ist, wobei der Ballon vorzugsweise aus dünnwandiger, nur wenig volumendehnbarer Polyurethanfolie hergestellt ist. In das urinableitende Lumen 2a des Katheterschaftes ist eine schlauchartige oder rohrartige Einführhilfe 4 eingesteckt. Die Spitze 5 des Katheterschaftes weist mindestens eine Drainageöffnung 6 auf. Bevorzugt können auch zwei oder mehrere Drainageöffnungen 6 vorgesehen sein, welche bevorzugt um jeweils gleiche Zwischenwinkel über den Umfang des Katheterschaftes 2 verteilt angeordnet sind. Beispielsweise könnten zwei Drainageöffnungen einander diametral gegenüber liegen, also um 180° versetzt sein, während drei Drainageöffnungen um 120°

**EP 4 025 286 B1**

gegeneinander versetzt wären. Die Spitze der Einführhilfe bzw. deren distale Öffnung 7 kommt im vollständig eingeschobenen Zustand unmittelbar proximal von der Drainageöffnung oder in direkter Deckung mit der Drainageöffnung zu liegen. Vorzugsweise schließt die Spitze der Einführhilfe derart passgenau, beispielsweise in einer konisch dichtenden oder rastend dichtenden Weise, mit der Innenwandung des Drainagelumens des Katheters ab, dass die zuführende Substanz bei der Applikation bzw. Injektion durch das Lumen der Einführhilfe nicht in retrograder Weise in den proximalen Teil des Drainagelumens des Katheters bzw. in den sich proximal anschließenden Spaltraum zwischen der Einführhilfe und der Innenwandung des Katheters entweichen kann.

Am proximalen Ende der Einführhilfe befindet sich in der bevorzugten Ausführung ein reservoirartiger, bevorzugt faltenbalgartiger Reservoirbehälter 8, der die zuzuführende Substanz aufnimmt und aus dem die Substanz vorzugsweise durch Ausdrücken oder Ausquetschen des Behälters zur Spitze des Katheters hin befördert wird. Der Behälter geht in seiner bevorzugten Ausführung bei Wirkung einer axial stauchenden oder alternativ auch radial quetschenden Kraft in einen axial bzw. radial rastenden Zustand über, der eine elastische Wiederaufrichtung des Behälters bei nachlassender wirkender Kraft verhindert. Einer Aspiration bereits applizierten Gels zurück in den Behälter kann so vorgebeugt werden.

Zur verbesserten Fixierung der relativen Position von Katheter und Einführhilfe verfügt der distale Teil des Behälters 8 über ein konusartiges Segment 9, das sich passgenau und optional rastend in den Konnektor 10 des Katheters einschieben lässt.

Abb. 2 zeigt eine Ausführung einer Einführhilfe 4, mit aus- oder angeformter Spitzenformation 7a, wobei diese beispielsweise aus weichem, plastisolartigem Material bestehen kann und dem relativ zur Spitze härterem Schaft 11 der Einführhilfe für eine möglichst atraumatische Passage der Harnröhre endständig aufsitzt, Die Spitze 7a weist bevorzugt ein endständig mündendes Lumen 12 auf, durch welches in Verlängerung des ebenfalls mit einem Lumen versehenen Schaftes 11 der Einführhilfe eine lubrifizierende Substanz in die Harnröhre abgegeben werden kann.

Die Spitze 7a der Einführhilfe kann im Übergang zum distalen Ende 5 des Katheterschaftes eine besondere, wulstartige Verdickung 7b aufweisen, die sich unter axialem Zug elastisch verschlankt, und so bei der Retraktion aus der relativen Position im Übergang zur Katheterspitze, herausgleitet. Die Wulst 7b kann optional mit längsverlaufenden, streifenartigen Schwächungen versehen werden, die bei der Überwindung einer bestimmten Zugkraft zum Kollaps der Wulst führen und so die Retraktion der wulstartigen Formation durch das Lumen des Katheters erleichtert. Die Wulst 7b formt im Verbund mit der vorderen Öffnung 5 des Katheters einen möglichst stufenlosen, atraumatischen Übergang. Die Wulst ermöglicht ferner, dass Substanz, welche bei der Katheteranlage in die Harnröhre appliziert wird, vor dem distalen Katheterende 5 gestaut und vor dem Katheterende hergeschoben werden kann, so dass dieses bei der urethralen Passage des Katheters im Bereich vor der Katheterspitze in ausreichender Menge verfügbar bleibt. Insbesondere bei der Applikation von Gelen kann so eine optimal lumenaufrichtende Wirkung bei der Einführung des Katheters in die Harnröhre erreicht werden.

Abb. 2a. Bei Verwendung härterer Materialien oder auch von Materialien mit höherer elastischer Rückstellkraft, kann die Spitze 7 der Einführhilfe 4 auch mit einem wellig ausgeformten Profil 7c versehen sein, die die Spitze flexibel und allseitig axial biegbar macht, das Lumen 11 der Einführhilfe aber erhält, Bei der Katheterisierung durch die Harnröhre können durch die wellige Profilierung besonders vorteilhafte, atraumatische Einführeigenschaften erreicht werden.

Abb. 2b. Ein entsprechend gewelltes Profil 7c kann auch im Bereich der Katheterspitze 5 des Blasenkatheterschaftes ausgeformt werden. In situ platziert, kann die Spitze in eine nach lateral gebeugte Position P abknicken, wodurch eine direkte, lanzierend wirkende Irritation und bei Langzeitanlage ulzerierende Läsion des der Spitze aufliegenden Blasendachs vermieden wird.

Abb. 2c zeigt eine besondere Ausführung der Einführungshilfe 4, bei der sämtliche funktionellen Segmente, inklusive des substanzaufnehmenden Behälters 8 am proximalen Ende, aus einem einzigen Rohling, vorzugsweise durch Blasformung aus einem schlauchförmigen Grundkörper ausgeformt werden. Der distale Anteil der Einführungshilfe kann optional mit einer, die axiale Verbiegung unterstützende Wellung versehen sein. Ferner können auch proximale Schaftanteile der Einführhilfe in bestimmten, für die erleichterte urethrale Passage vorteilhaften Abschnitten mit einem entsprechenden, wellig ausgeformten Profil versehen sein. Zusätzlich kann die Einführhilfe im proximalen Segment durch ein die lagesichernde Passung in den Blasenkatheterkonnektor 10 unterstützendes, kongruent passgenau ausgeformtes Segment 9 versehen sein. Alternativ zur vollständigen Blasformung aus einem einzigen Rohling kann die erfindungsgemäße Einführhilfe anteilig oder vollständig auch durch Spritzguss, Guss oder auch durch Tauchverfahren hergestellt werden.

Die Einführhilfe besteht vorzugsweise aus einem Material mit guten Gleiteigenschaften, was für die komfortable Retraktion aus dem Verweilteil der Vorrichtung entscheidend ist. Zur Erreichung dieser Gleitfähigkeit kann das verwendete Basismaterial beispielsweise mit einem Wachszusatz versehen werden, der auf der Oberfläche einen den Reibungswiderstand reduzierenden Film ausbildet.

Abb. 2d zeigt eine abgewandelte Ausführung des in Abb. 2c vorgestellten Bautyps, wobei der Reservoirbehälter 8a eine in sich abgeschlossene, baulich mit der Einführhilfe 4 unverbundene Einheit darstellt, die in einen konnektorartigen Fortsatz 9a aufweist, der passgenau in einen formkongruenten Aufnahmetrichter 9b eingesteckt wird. Das apikale Ende des Behälters weist eine besonders dünnwandige oder auch sonstig präformierte Fläche 9c auf, die beispielsweise durch eine dornartige Verlängerung 9d des Schaftanteils, welche in den Aufnahmetrichter hineinreicht, beim Einschub des Behälters perforiert wird, und so den Übertritt von Substanz in die für die Anwendung vorbereitete, gesteckte Kombination von Katheter und Einführhilfe erlaubt. Die Ausführung ist insbesondere für Substanzen erforderlich, die besondere Sterilisationsmethoden erfordern, die von der Sterilisation des eigentlichen Kathetersets abweichen, wie dies beispielsweise für gelartige Verbindungen der Fall sein kann. Der Behälter kann somit dem Katheterset separat beigefügt werden und erst unmittelbar vor dem Katheterisieren in das Endstück der Einführhilfe eingesteckt werden.

Abb. 3a zeigt eine besondere Ausführung des transurethralen Schaftsegmentes 12 des Katheters. Zur Gewährleistung eines möglichst großen Drainagedurchmessers im Inneren des Schaftes wird die Schaftwandung bevorzugt aus dünnwandigem PUR, PVC oder auch PVC/PUR Blend-Material ausgeführt. Um die dünnwandigen Schaftstrukturen zu stabilisieren und einem eventuellen Lumenverschluss durch Knickung oder Torsion des Schaftes zu vermeiden, schlägt die Erfindung ein welliges Profil 13 der Schaftwandung in Art eines Wellschlauches vor. Die wellige "Korrugation" des Schaftes macht den jeweiligen Schlauch biegsam und reduziert eventuelle, durch die Biegung verursachte elastische Rückstellwirkungen bzw. durch diese Rückstellwirkungen auf die exponierten Gewebe dauerhaft wirkende Kräfte, Insbesondere bei der Verwendung von PUR ist der erreichbare, knickungsfreie Biegeradius des Katheterschaftes ohne entsprechende Profilierung des Schaftes eingeschränkt, PUR weist zudem, im Gegensatz zu PVC, bei der Biegung eine deutlich höhere elastische Rückstellwirkung auf. Die beschriebene Korrugation 13 des Katheterschaftes kann durchgängig über dessen gesamte Länge aufgebracht oder auch nur in bestimmten Bereichen aufgebracht sein, in denen sich in situ platziert ein besonders kleiner Biegeradius bei entsprechend geringer elastischer Spannung einstellen soll.

Beispielsweise kann der wellig ausgeformte Schaft folgende Merkmale aufweisen: Außendurchmesser 6,0 mm, Innendurchmesser 5,4 bis 5,9 mm bevorzugt 5,6 bis 5,8 mm, Polyurethan der Härte Shore 85A oder 90A, Wellungsamplitude 1,0 bis 1,5 mm, peak-to-peak Abstand der Wellung 0,5 bis 1,5 mm.

Abb. 3b zeigt einen besonders dünnwandig ausgeführten transurethralen Schaftschlauch 14 der Verweileinheit 2, dessen Wandung annähernd auf Folienstärke von beispielsweise 0,05 mm reduziert ist. Ein damit erzielbarer Vorteil ist, dass ein derart dünner Schaftschlauch 14 sich dem Verlauf der Harnröhre optimal anpassen kann, so dass auch bei einer längeren Anwendungsdauer von beispielsweise einer oder mehreren Wochen keine Verletzungen der Harnröhren-Innenwand zu befürchten sind.

Zur Vermeidung von lumenverschließenden Knickungen und Torsionen der Schlauchfolie 14 wird diese zum Beispiel mit einem vorzugsweise frei innenliegenden, stab-, rohr- oder schlauchartigen Element SE1 ergänzt, welches in Fig. 3b oben dargestellt ist und das intra-vesikale Segment VS des Katheters über die Länge der Harnröhre hinweg mit dem extrakorporalen Konnektorteil KO in entsprechend stabilisierender Weise verbindet. Im Falle einer schlauchartigen Ausführung des Elementes SE1 kann dessen Lumen zur Füllung des retinierenden Ballons verwendet werden.

Andererseits kann zur Stabilisierung eines schlauchfolienartig dünnwandigen Schaftschlauches 14 dessen Wandung auch durch eine beispielsweise netzartige Struktur SE2 verstärkt sein, die vorzugsweise auf die Innenfläche des Schlauches 14 in flächig und fest verbundener Weise aufgebracht ist. Ein entsprechendes, netzartiges Geflecht SE2 kann auch zwischen zwei koaxial angeordnete Schlauchfolienlagen fest verbaut, beispielsweise mit diesen Lagen koextrudiert werden. Anstelle eines netzartigen Geflechts kann auch eine spiralförmige Anordnung verwendet werden, oder eine andere flächige Anordnung mit ausreichenden Selbstaufrichtungseigenschaften.

Eben diese Selbstaufrichtungseigenschaften verleihen der ansonsten sehr flexiblen Schlauchfolie 14 die Fähigkeit, sich Tendenzen zum Abknicken zu widersetzen und/oder eingetretene Torsionen der Schlauchfolie 14 zurückzuführen bzw. zurückzustellen. Beide Maßnahmen dienen dazu, das Lumen innerhalb der folienartigen Schlauchfolie 14 offenzuhalten.

Abb. 4 zeigt einen reservoirartigen Behälter 8, weicher die gut dosierbare Abgabe eines Gleitmittels in die Harnröhre gestattet. Der Behälter ist vorzugsweise als faltenbalgartiges Gefäß gestaltet, welches beim Zusammenschub des Balges durch Druck auf den Boden 15 des Behälters in der jeweiligen eingeschobenen Position einrastet, sich also bei Entlastung des Balgs nicht durch elastische Wirkung in sein aufgerichtetes Ausgangsmaß zurückstellt. Der Behälter kann farbliche Bereiche 16 tragen, die bestimmte Mengen der zu applizierenden Substanz entsprechen. Werden die markierten Segmente gestaucht bzw. in Summe kollabiert, ist das den markierten Segmenten entsprechende Volumen freigesetzt.

Abb. 5 zeigt den vorderen Abschnitt eines Blasenkatheters mit darin positionierter Einführhilfe 4 für die Applikation einer lubrifizierenden und/oder lumenaufrichtenden Substanz. Um das Austrocknen der in der Einführhilfe bevorrateten Substanz zu verhindern, ist die vordere Austrittsöffnung 12 des Einführelementes daher mit einem Verschlusselement 17 versehen, welches die Öffnung 12 der Einführhilfe hermetisch dicht verschließt. Der untere, die Öffnung verschließende Teil des Elementes 17 ist von einer tüten- bzw.- fingerlingartigen Folienhülle 18 umgeben, die den Katheterschaft mit einem gewissen Spaltraum SR umgibt. Die Folienhülle weist bevorzugt eine Länge von ca. 8 bis 12 cm auf. Wird der Verschluss 17 aus der Austrittsöffnung der vorderen Öffnung der Einführhilfe entfernt, kann durch Kompression des Reservoirbehälters Gel in den Spaltraum eingespritzt werden, wobei es sich dort gleichförmig verteilt. Die für die Füllung des Spaltraumes erforderliche Menge an Gel kann beispielsweise durch eine entsprechende Farbmarkierung von Segmenten des Reservoirbehälters vorgegeben werden. Der Anwender kollabiert das markierte Segment durch axial wirkenden Daumendruck auf den Balgboden, und fördert so das nötige Volumen in den Spaltraum.

Neben einer farblichen Markierung von Faltenbalgsegmenten können differenziert ausgeführte Abschnitte des Reservoirbehälters, insbesondere von differenziert ausgestalteten Segmenten eines Faltenbalgs, eine taktil erfassbare bzw. orientierte Teilmengenfreisetzung ermöglichen. Beispielsweise können die proximalen, dem Balgboden unmittelbar benachbarten Balgfalten in Relation zu den distalen Balgfalten weicher bzw. mit geringerem Widerstand stauchbar ausgeführt sein, so dass der Anwender nach einer initialen Kompression des Faltenbalgs zur Freisetzung einer ersten Teilmenge der lubrifizierenden und/oder lumenaufrichtenden Substanz aus dem proximalen Segment des Balges, beim Übergang in das distal angrenzende Faltenbalgsegment einen tastbaren, erhöhten Widerstand wahrnimmt, welcher ihm signalisiert, dass nun die erste Teilmenge freigesetzt ist und vor der Freisetzung der zweiten Teilmenge der lubrifizierenden und/oder lumenaufrichtenden Substanz zunächst das distale Ende der Kathetervorrichtung in die Harnröhre eingeführt werden soll.

Unmittelbar vor dem Einführen des Katheters in die Harnröhre wird die Haube dann, mittels des an ihr apikal befestigten Verschlusselementes nach frontal vom Katheter abgezogen. Zur Vermeidung einer nichtbeabsichtigten Einführung der Folienhaube in das Ostium ist die Haube apikal vorzugsweise mit einem etwa daumengroßen, beispielsweise blattähnlichen Fortsatz versehen, der zudem das Entfernen bzw. Abziehen der Kappe erleichtert.

Abb. 6 zeigt einen optionalen, faltenbalgartigen Behälter 19 zum Befüllen des Retentionsballons mit vorzugsweise Luft, wobei der Faltenbalg ebenfalls bevorzugt über eine rastende Faltung verfügt, die gewährleistet, dass sich der Balg 19 bei Entlastung nicht elastisch aufrichtet und somit das Füllmedium wieder aus dem Retentionsballon absaugt. Der Balg 19 kann zur Bemessung der Luftmenge mit Farbmarkierungen 20 versehen sein, die den einzustellenden Füllzustand des Retentionsballons entsprechend vorgeben, so dass dieser beispielsweise partiell mit ca. 80% seines freien Ballonvolumens befüllt werden kann, eine vollständige Füllung anzeigt, oder auch eine Füllung herstellt, die den Ballon in einen moderat, beaufschlagten, gedehnten Zustand überführt, Der Blasenkatheter ist dabei entsprechend mit einem Verschluss- oder Ventilelement zur Füllung des Retentionsballons ausgestattet, dass im Anschluss an die Befüllung eine selbstdichtende Entkoppelung des Behälters vom Katheter gestattet, Der Anschlusskonus 21 des Behälters ist bevorzugt so entworfen, dass er im aufgesteckten Zustand das Ventil öffnet und eine Befüllung erlaubt. Wird der Konus aus dem Ventil abgezogenen, schließt sich das Ventil.

Abb. 7. Die abgebildete U-förmige Konfiguration der hier dargestellten Katheterset-Vorrichtung S, bestehend aus einem erfindungsgemäßen Blasenkatheter 1, einer in das drainierende Lumen des Katheters eingeschobene Einführhilfe 4, sowie angekoppelten Reservoirbehältern 8,19, wird durch eine, der Handfläche des Anwenders in seiner Form angepasste Haltevorrichtung 22 gewährleistet, die zum einen das proximale, konnektorseitige Ende des Katheters aufnimmt und fixiert, zum anderen den distalen Schaftanteil des Katheters greift. Die Haltevorrichtung verfügt optional über ein palmares Griffstück 23 für das handhabungssichere Greifen der Haltevorrichtung. Der daumenseitige Anteil der Haltevorrichtung DH nimmt den proximalen Teil der Katheteranordnung auf. In bevorzugter Ausführung werden die Konnektoranteile der Katheteranordnung in passgenauen Präformationen des Griffstücks bzw. der Haltevorrichtung in beispielsweise rastender Weise fixiert. Die Fixierung ermöglicht dabei eine ausreichende Widerlagerfunktion, so dass beispielsweise durch Daumendruck auf den Reservoirboden Gel zur Katheterspitze hin befördert werden kann, ohne dass durch den anlastenden Druck des Daumens eine Herauslösung der Konnektoranteile aus der Fixierung verursacht wird. Andererseits muss nach Anlage des Katheters bzw. seiner Sicherung in der Blase der gesamte proximale Konnektorteil vom Anwender mit angemessenem Widerstand aus der rastenden Position entnommen werden können. Vorteilhaft sind hierbei den Katheterschaft bzw. die Konnektoranteile aufnehmende C-förmige Tiefziehungen. Die jeweiligen Anteile der Vorrichtung rasten dabei in eine vorzugsweise elastisch verformbare Haltespange ein. Durch konvexe Verformung bzw. Wölbung der Haltevorrichtung, beispielsweise durch Quetschung der Haltespange in der palmaren Hand, kann das C-förmige Profil der Tiefziehung geöffnet, und der Katheter so freigegeben werden.

Insbesondere der Gel-Behälter 8 liegt derart in der Spange bzw. im Griffstück positioniert, dass er vom Anwender durch Daumendruck leicht gestaucht bzw. komprimiert werden kann. Benachbart vom Gel-Behälter kann der Befüllbehälter für die Blockung des Ballons angeordnet sein, Er kann in entsprechender Weise durch Daumendruck gestaucht oder ausgepresst werden. Eine ballonartige, mit Luft befüllte Ausführung, kann zum Beispiel in einer löffelartigen Mulde 24 der Spange oder des Griffes gebettet sein und dort quasi einem Widerlager aufliegen.

Zur Gewährleistung des Schutzes vor Kontamination des Katheters bei der Katheteranlage ist die Haltevorrichtung mit einer dünnwandigen, beutelartigen Folie 27 ausgestattet. Die Beutelfolie schützt dabei insbesondere den Katheterabschnitt, der von der greifenden Pinzette oder Zange 25 bis zum Konnektor der Kathetervorrichtung reicht. Der schützende Beutel ist für die Aufnahme des geschlungenen, mittigen Katheterschaftes gestaltet. Der Folienschutz ermöglicht es dem Anwender auf die Anlage steriler Handschuhe zu verzichten und den erfindungsgemäß ausgerüsteten bzw. in der Haltevorrichtung fixierten Katheter steril einzubringen,

Abb. 7a beschreibt eine besondere, pinzettenartige oder zangenartige Klemmung 25 die seitlich an die Haltevorrichtung 22 angebracht ist. Wird die Wippe 26 mit Daumendruck belastet, greift die Klemmung den einzuführenden Katheterschaft 2. Wird die Wippe entlastet, kann die Klemmung in eine neue relative Position zum Katheterschaft versetzt werden. Die wippen- oder zangenartige Klemmung ist bevorzugt derart ausgeführt, dass die greifenden Elemente klauenartig gestaltet sind und die Klauen dabei einen gewissen Überstand 36 aufweisen, der den Katheter im nichtbelasteten Zustand der Zange zwar innerhalb der Klaue axial beweglich macht, die vollständige Freigabe des Katheters aus der Haltevorrichtung aber verhindert, und erst bei entgegen gerichteter Betätigung der Wippe ermöglicht.

Sind die etwa vorderen zehn Zentimeter des mit lubrifizierender Substanz benetzten Katheterschaftes in die Harnröhre eingeführt, wird der Katheterschaft in der Klemmung durch Entlastung der Wippe freigegeben, und diese dann nach proximal hin auf dem Schaft versetzt, wo die Klemmung den Schaft bei Druck auf die Wippe erneut greift. Der Katheterschaft kann so vom Anwender sukzessive, in der Art einer pinzetten- oder zangenartigen Führung in die Harnröhre vorgeschoben werden. Sind etwa zwei Drittel der Schaftlänge in die Harnröhre eingeführt, wird der Katheter durch Befüllen des Ballons gesichert und der Katheter aus den fixierenden Tiefziehungen oder Formationen der Haltevorrichtung vollständig entnommen.

Abb. 8 zeigt eine besondere Ausführung eines Lochtuches 28, welches aus zwei Lagen eines bevorzugt vliesstoffartigen Materials besteht, wobei die beiden Lagen im Bereich des zentral angeordneten Lochs 29 durch einen Saum 30 mittels Klebung oder Verschweißung miteinander dicht schließend verbunden sind. Der Anwender nimmt das Tuch auf, indem er mit der C-förmig geöffneten Hand in den Zwischenraum Z zwischen beiden Lagen hineingreift und das Loch 29 schließlich umgreift. Die direkte Führung des Loches durch C-förmiges Umgreifen mit Daumen und Zeigefinger erlaubt es, die Öffnung in optimal modellierender Weise an die zu reinigenden, das Orificium umgebenden Flächen heranzuführen bzw. den Penis optimal unterstützend zu greifen oder die Labien zu spreizen. Alternativ zu einem Zwischenraum Z kann das Lochtuch 28 auf der dem Patienten zugewandten Seite 28a mit einem großzügig dimensionierten, stilisiert zugeschnittenen, handschuhartigen Element 31 ausgestattet sein, dessen Rückenseite mit der patientenzugewandten Seite 28a flächig verbunden ist. Als weitere, weniger bevorzugte Ausführungsvariante kann das Element 31 auf der palmaren Seite mit der patientenabgewandten Seite 28b des Lochtuches verbunden sein. Das Lochtuch kann zum Umfang des Tuches hin, vom Loch ausgehend mit einem durchgehenden Schlitz 32 versehen sein. Das Tuch kann ferner, bevorzugt in einer Linie oberhalb der ulnaren Kante 33 der das Lochtuch aufnehmenden Hand angeordnet, mit blisterartigen, für die Einzelaufnahme von getränkten Tupfern präformierten Kompartimenten 34 ausgestattet sein. Der Blister ist mit einer aufreißbaren Folie 35 zum Anwender hin verschlossen. Die einzelnen Tupfer werden durch an einem mit Schutzfolie eingehausten, stempelartige Ausläufer vom Anwender gegriffen und dem Blister so entnommen.

Die Erfindung beschreibt ferner ein Verfahren zur Katheterisierung der Blase eines Patienten, wobei die Anzahl der erforderlichen Komponenten für den Reinigungsund Einführungsprozess auf ein Mindestmaß reduziert ist. Die erfindungsgemäß beschriebene, anwendungsfertig vorbereitete Kathetervorrichtung wird hierzu vorteilhaft mit einem handschuhartig gestalteten, den Penis direkt greifenden oder die Labien spreizenden Lochtuch 28 kombiniert, dass wie die Kathetervorrichtung in besonderer Weise für eine einhändige Handhabung ausgelegt ist. Im Verbund der beiden Funktionseinheiten, Handschuh-Tuch und Kathetervorrichtung, kann der Anwender den gesamten Katheterisierungsvorgang ohne assistierende Person durchführen.

Beide Funktionseinheiten sind gemeinsam in einem Set verpackt. Nach Öffnung der Umverpackung des Sets greift der Anwender mit seiner Hand direkt in die handschuhartig gestaltete Lochtucheinheit und nimmt diese so aus dem Set auf. Der Anwender greift dann direkt den Penis oder spreizt die Labien. Er erreicht so eine optimal schützende Position des Tuchs zum zu reinigenden und dann zu katheiterisierenden Orificium, Er faltet dann die lateralen Anteile des Tuchs nach Bedarf auf und befestigt diese am Bauch, den Schenkeln oder am Gesäß des Patienten, durch entsprechende selbstklebende Flächen.

Als Besonderheit weist das Handschuh-Tuch 28 optional in das Tuch integrierte, blisterartig tiefgezogene Folienkompartimente 34 auf, die durch Aufreißen oder Abziehen einer Schutzfolie 35 mit der freien Hand geöffnet werden, und anwendungsfertige, mit Antiseptikum getränkte Tupfer für die Haut- und Schleimhautreinigung enthalten. Die Tupfer werden an einer stempelgriffartigen Formation aus dem Blister herausgegriffen, die durch geschlossen tiefgezogene Folie geschützt ist und so den direkten, verschmutzungsfreien Griff des Anwenders erlaubt. Die Tupfer können dann auf das Lochtuch bzw. in eine daran angebrachte taschenartige Formation abgeworfen werden.

Nach durchgeführter Reinigung greift der Anwender dann die Kathetervorrichtung mit der freien Hand und führt den mit Gel lubrifizierten, durch die Einführhilfe stabilisierten, distalen Katheteranteil, durch die Einführhilfe verstärkt und die Haltevorrichtung lanzenartig geführt, in die Harnröhre ein. Er appliziert dann durch Daumendruck die noch im Reservoirbehälter verbliebene Substanz in die Harnröhre und schiebt den Katheter durch sukzessives Greifen und Lösen des Katheterschaftes mit der Klemme der Haltevorrichtung in die Blase vor. Während des Einführens des Katheters justiert der Anwender den Penis durch das Lochtuch durch direkten Griff in der jeweils optimalen Position bzw. hält die Labien durch digitale Spreizung optimal offen.

Sind die beiden vorderen Drittel des Katheters in die Harnröhre eingeführt, wird der Ballon des Katheters durch Ausdrücken des Befüllbehälters mit dem Daumen geblockt. Anschließend wird die Hand aus der Handschuh-Tuchkombination entfernt und der proximale Katheteranteil aus der Haltevorrichtung retrahiert.

Die Einführhilfe wird dann aus der Vorrichtung retrahiert und der Befüllbehälter vom Katheterventil entfernt. Der Katheter wird dann mir einer urinableitenden Schlauch-Beuteleinheit verbunden.

Die retinierende Ballonkomponente des Blasenkatheters wird vorzugsweise durch Blasformung aus einem zuvor, in einem separaten Herstellungsschritt geblasenen Polyurethan-Schlauchrohling ausgeformt, vorzugsweise im Durometer-Bereich Shore 80A bis 95A, besonders bevorzugt zwischen 85A und 90A. Die Wandungsstärke liegt bevorzugt bei 5 bis 25 μm, besonders bevorzugt bei 7 bis 12 μm. Bei Verwendung von Materialtypen wie beispielsweise PUR der Elastollan1100 Gruppe der Firma BASF oder PUR der Sorte Pellethane 2363 der Firma Lubrizol können durch Blasformung aus vorextrudiertem Material weichfolienartige Gebilde ausgeblasen werden, die in idealer Weise niedrigste Wandstärke mit hoher Formstabilität verbinden. Insbesondere bei hoher nach außen hin gerichteter axialer Zugwirkung gewährleisten mikro-dünne PUR-Ballonkomponenten eine zuverlässige Ankerwirkung.

Neben Polyurethan kann das Ballonelement, weniger bevorzugt, aus vergleichbaren Weichfolien alternativer Materialien, wie beispielsweise Polyethylen-basierte Mischungen oder TPE basierten Grundstoffen gefertigt werden. Für die Blasformung sind ferner koextrudierte Ausgangsmaterialien denkbar, die beispielsweise PUR und PVC in koaxial extrudierter Weise im Rohschlauch kombinieren.

Ebenfalls weniger bevorzugt können auch konventionelle sleeve-basierte, nicht vorgeformte, elastisch aufzudehnende Ballonelemente verbaut sein.

Bezugszeichenliste

| | | | | | |
|---|---|---|---|---|---|
| 1 | Vorrichtung | | 20 | Farbmarkierung | |
| 2 | Verweilteil, Katheter | | 21 | Anschlusskonus | |
| 2a | Spaltraum | | 22 | Haltevorrichtung | |
| 3 | Ballon | | 23 | Griffstück | |
| 4 | Einführhilfe | | 24 | löffelartige Mulde | |
| 5 | Spitze | | 25 | Zange, Klemmung | |
| 6 | Drainageöffnung | | 26 | Wippe | |
| 7 | Öffnung | | 27 | Schutztasche | |
| 7a | Spitze, Spitzenformation | | 28 | Lochtuch | |
| 7b | Wulst, Verdickung | | 28a | patientenzugewandte Seite | |
| 7c | welliges Profil | | 28b | patientenabgewandte Seite | |
| 8 | Reservoirbehälter | | 29 | Loch | |
| 8a | Reservoireinheit | | 30 | Saum | |
| 9 | Segment | | 31 | Element | |
| 9a | Anteil | | 32 | Schlitz | |
| 9b | trichterartige Ausformung | | 33 | Kante | |
| 9c | perforierrbare Fläche | | 34 | Folienkompartiment | |
| 9d | dornartige Verlängerung | | 35 | Folie, Schutzfolie | |
| 10 | Konnektor | | 36 | Überstand | |
| 11 | Schaft | | DH | daumenseitiges Halteteil | |
| 12 | Lumen | | KO | Konnektorteil | |
| 13 | Abschnitt, Profil | | P | lateral gebeugte Position | |
| 14 | Schaftschlauch | | S | Set, Katheter-Set | |
| 15 | Boden | | SR | Spaltraum | |
| 16 | Segment, Bereich | | SE1 | stabförmiges Element | |
| 17 | Element, Verschluss | | SE2 | Schlauchförmiges Element | |
| 18 | haubenartiges Element | | VS | intra-vesikales Sgment | |
| 19 | Balg, Behälter | | Z | Zwischenraum | |

**Patentansprüche**

1.    System zur Katheterisierung der Blase eines Patienten, umfassend eine Vorrichtung (1) und eine Substanz, wobei die Vorrichtung

    a) ein schaftförmiges, in der Blase und in der Harnröhre nach Art eines Katheters platzierbares, drainierendes Verweilteil (2) mit einem urinableitenden Lumen (2a) und wenigstens einer Drainageöffnung (6) im Bereich der distalen Spitze (5) des schaftförmigen Verweilteils (2); sowie

b) eine in das urinableitenden Lumen (2a) des schaftförmigen Verweilteils (2) eingesteckte, retrahierbare, die urethrale Einführung und Passage erleichternde Einführhilfe (4) von rohr- oder schlauchartigem Aufbau mit einer apikalen Öffnung (7) zur endständigen Freisetzung der Substanz durch die apikale Öffnung (7) im Bereich der distalen Spitze des Verweilteils (2) umfasst;

**dadurch gekennzeichnet, dass**

c) die durch die apikale Öffnung (7) freigesetzte Substanz lubrifizierende und/oder lumen-aufrichtende Eigenschaften hat; und

d) der das urinableitenden Lumen (2a) umgebende Schaftanteil des Verweilteils (2) sich von einem Retentionsballon (3) bis zu einem proximalen Konnektor (10) erstreckt.

2. System nach Anspruch 1, **dadurch gekennzeichnet, dass** sich die Substanz aus der apikalen Öffnung (7) der Einführhilfe (4) heraus durch eine ableitende Öffnung (6) im Bereich der Katheterspitze derart gedichtet applizieren lässt, dass ein Rückstrom der Substanz in den sich proximal an die Öffnung der Einführhilfe anschließenden Spaltraum (2a) zwischen Einführhilfe und dem drainierenden Katheterlumen zuverlässig vermieden ist.

3. System nach Anspruch 1 oder 2, **dadurch gekennzeichnet, dass** sich die distale Öffnung (7) der Einführhilfe (4), unmittelbar benachbart zur distalen, drainierenden Öffnung (6) des Verweilteils (2), in das drainierende Lumen des Verweilteils (2) hinein öffnet oder sich in einer direkten Deckung bzw. gemeinsamen Flucht mit der drainierenden Öffnung (6) des Verweilteils (2) befindet.

4. System nach einem der Ansprüche 1 bis 3, **dadurch gekennzeichnet, dass** sich die Spitze (7a) der Einführhilfe (4) über die distale, drainierende Öffnung (6) des Verweilteils (2) hinaus erstreckt, vorzugsweise wobei die Spitze (7a) der Einführhilfe (4)

a) eine gekrümmte Vorformung für die erleichterte urethrale Passage aufweist, und/oder

b) für eine erhöhte Flexibilität, insbesondere wellig, präformiert ist, um eine flexible Biegung der Spitze (7a) der Einführhilfe (4) zu ermöglichen, welche Biegsamkeit bevorzugt über 90 Grad, oder im idealen Falle über 135 Grad hinaus reicht, und/oder

c) im Übergangsbereich zur distalen Öffnung (6) des Verweilteils (2) eine den Übergang glättende wulst- oder bulbus- oder spindelförmige Querschnittserweiterung (7b) aufweist, die sich unter Zugwirkung von proximal her derart verformt, dass die Querschnittserweiterung (7b) durch das Lumen des vorzugsweise katheterförmigen Verweilteils (2) herausgezogen werden kann.

5. System nach einem der vorangehenden Ansprüche, **dadurch gekennzeichnet, dass** die Einführhilfe (4) im Bereich eines Konnektors (10) des Blasenkatheters oder Verweilteils (2) durch leichte Pressung oder Rastung in ihrer Position gesichert werden kann, und hierzu einen passgenau, formkongruent ausgeformten Bereich (9) aufweist.

6. System nach einem der vorangehenden Ansprüche, **dadurch gekennzeichnet, dass** die Einführhilfe (4)

a) einen distalen Bereich aufweist, der aus einer rohr- oder schlauchartigen, optional die Vorrichtung (1) versteifenden Verlängerung besteht, aus der bevorzugt direkt, in einteiliger Bauweise, eine substanzaufnehmende Reservoireinheit (8) hervorgeht, und/oder

b) einen proximalen Bereich aufweist, der eine trichterartige Ausformung (9b) aufweist, in die eine baulich separate Reservoireinheit (8;8a) über deren formkongruent ausgeformten Anteil (9a) dicht schließend eingeschoben werden kann, insbesondere wobei die eingeschobene Reservoireinheit (8;8a) am distalen Ende eine perforierbare Fläche (9c) aufweist, die durch eine dornartige Verlängerung (9d) des distalen Bereichs der Einführhilfe (4) bei vollständiger, endständiger Einführung des Reservoirs (8;8a) eröffnet werden kann.

7. System nach einem der vorangehenden Ansprüche, **gekennzeichnet durch** eine Reservoireinheit (8;8a) aus einem balgartig ausgeformten Material, das sich bei Kompression des Balges bevorzugt in der jeweiligen Position in rastender Weise fixiert und eine Wiederaufrichtung des Balges verhindert, vorzugsweise wobei die Reservoireinheit (8;8a)

a) farbliche markierte Segmente (16) aufweist, wodurch bei deren einrastender Kompression die Abgabe eines bestimmten Volumens kodiert werden kann, und/oder

b) Segmente (16) aufweist, die bei der manuellen Kompression jeweils spezifische Faltungs- und/oder Kollapswiderstände entwickeln, und so einen taktil fühlbaren Übergang von einer ersten, zu dosierenden Volumenmenge zu einer nachfolgenden, zweiten Volumenmenge ermöglichen.

8. System nach einem der vorangehenden Ansprüche, **dadurch gekennzeichnet, dass** der Schaftanteil des Verweilteils (2) in einem oder mehreren Abschnitten (13) des Schaftes wellig präformiert ist, wodurch die axiale Biegung des Schaftes erleichtert und elastisch wirkende Rückstellkräfte im Schaft reduziert werden können.

9. System nach einem der vorangehenden Ansprüche, **dadurch gekennzeichnet, dass** der Schaftanteil im urethralen Abschnitt oder im Abschnitt zwischen dem Retentionsballon (3) und dem proximalen Konnektor (10) des Verweilteils (2) aus folienartig dünnwandigem Schlauchmaterial (14) besteht, vorzugsweise umfassend

a) eine oder mehrere, im Innenlumen der Schlauchfolie (14) vorzugsweise frei liegend angeordnete, stab- oder rohrartige Struktur(en) (SE1), insbesondere um den Verschluss des Drainagelumens infolge axialer Verwindungen oder Knickungen der Schlauchfolie (14) zu vermeiden, und/oder

b) eine mit der Schlauchfolie (14) fest verbundene oder in diese integrierte, netzartige Armierung (SE2), die das das Lumen der Schlauchfolie (14) elastisch aufrichtet, insbesondere um axiale Verwindungen zu erschweren und/oder stattgefundene Verwindungen wieder in die nicht-torquierte Ausgangslage zurückzuführen.

10. System nach einem der vorangehenden Ansprüche, **dadurch gekennzeichnet, dass** der distale Bereich des Verweilteils (2) und/oder der Einführhilfe (4) mit einem haubenartigen Element (18) versehen ist, vorzugsweise wobei der Spaltraum (SR) zwischen der Haube (18) und dem Verweilteil (2) durch eine in den Spaltraum (SR) zugeführte, insbesondere lubrifizierende und/oder lumenaufrichtende Substanz befüllbar ist.

11. System nach einem der vorhergehenden Ansprüche, **dadurch gekennzeichnet, dass** die Haube (18) apikal ein mit der Haube (18) fest verbundenes, beispielsweise zapfenartiges Verschlusselement (17) aufweist, das die Austrittsöffnung der Einführhilfe (4) propfenartig verschließt, und welches nach dessen Lösung von der Einführhilfe (4) das Freisetzen der lubrifizierenden und/oder lumenaufrichtenden Substanz aus dem Binnenlumen der Einführhilfe (4) sowie dessen endständiger, reservoirartiger Erweiterung (8;8a) ermöglicht.

12. System nach einem der vorangehenden Ansprüche, **dadurch gekennzeichnet, dass** die aus dem Verweilteil (2) und einer in das Verweilteil (2) eingesteckten Einführhilfe (4) bestehende Einheit in einer Haltevorrichtung (23,25) eingelegt und dort reversibel fixiert ist, und dabei in eine U-förmige, schlingenartige Konfiguration genommen wird, wobei der distale Teil der Einheit (2,4) durch eine zangen- oder wippenartig öffnende und schließende Vorrichtung (25) gehalten wird, und der proximale, den Konnektor (10) tragende Bereich der Einheit (2,4) in der Haltevorrichtung (25) derart in bevorzugt C-förmigen Aussparungen aufgenommen ist, dass ein in den oder an dem Konnektorteil (10) ein- oder angesteckter Befüllbalg oder -behälter (19) und/oder ein dort gegebenenfalls ein- oder angesteckter Reservoirbehälter (8;8a) durch Daumendruck komprimiert bzw. entleert werden kann.

13. System nach Anspruch 12, **dadurch gekennzeichnet, dass** der proximale Bereich des Verweilteils (2), vorzugsweise etwa die proximale Hälfte des Verweilteils (2), insbesondere etwa die proximalen zwei Drittel des Verweilteils (2), in einer umschließenden folienartigen Schutztasche (27) aufgenommen ist, die vorzugsweise fest oder auch abziehbar mit der das Verweilteil (2) aufnehmenden Haltevorrichtung (23,25) verbunden ist.

14. System nach einem der Ansprüche 12 oder 13, **dadurch gekennzeichnet, dass** die zangen- oder wippenartige Vorrichtung (25) in dem sich öffnenden lateralen Teil ein überbiss-artiges Greifen (36) des Schaftes des Verweilteils (2) ermöglicht, welches verhindert, dass sich der Schaft (2) des Verweilteils (2) beim Lösen des Verweilteils (2) aus dem Griff der Zange (25) heraus bewegen kann und somit aus der Haltevorrichtung (25) freigesetzt wird, und deren Überbiss (36) sich erst bei einer maximalen Öffnung der Wippe (26) öffnet und dadurch den Katheterschaft (2) freigibt.

15. System nach einem der vorhergehenden Ansprüche, **dadurch gekennzeichnet, dass** das Material des Verweilteils (2) eine Shorehärte (x) aufweist, und dass das Material der Einführhilfe (4) eine Shorehärte (y) aufweist, wobei die Shorehärte (y) des Materials der Einführhilfe (4) der Shorehärte (x) des Materials des Verweilteils (2) entspricht oder jene überschreitet, also:

$$y = x,$$

oder:

$$y > x.$$

**Claims**

1. A system for a catheterization of the bladder of a patient, comprising a device (1) and a compound, wherein the device comprises

   a) a shaft-shaped, draining indwelling part (2) that can be placed in the bladder and in the urethra in the manner of a catheter, having a urine draining lumen (2a) and at least one drainage opening (6) in the region of the distal tip (5) of the shaft-shaped indwelling part (2); and
   b) a retractable insertion aid (4) that is inserted into the urine draining lumen (2a) of the shaft-shaped indwelling part (2), which facilitates insertion into and passage through the urethra and has a tubular or hose-like construction with an apical opening (7) for terminal release of a substance in the region of the distal tip of the indwelling part (2);
   **characterized in that**
   c) the substance which is released through the apical opening (7) has lubricating and/or lumen-straightening properties; and
   d) the shaft portion of the indwelling part (2) which encompasses the urine draining lumen extends from a retention balloon (3) up to a proximal connector (10).

2. The system according to claim 1, **characterized in that** the substance can be applied from the apical opening (7) of the insertion aid (4) through a draining opening (6) in the region of the catheter tip in such a sealing manner that a reverse flow of the substance into the gap area (2a) between the insertion aid and the draining catheter lumen, which gap area is proximally adjacent at the opening of the insertion aid, is reliably prevented.

3. The system according to claim 1 or 2, **characterized in that** the distal opening (7) of the insertion aid (4) opens into the draining lumen of the indwelling part (2) directly adjacent to the distal draining opening (6) of the indwelling part (2), or is located in a direct coincidence or common alignment with the draining opening (6) of the indwelling part (2).

4. The system according to one of the claims 1 to 3, **characterized in that** the tip (7a) of the insertion aid (4) extends beyond the distal, draining opening (6) of the indwelling part (2), preferably whereby the tip (7a) of the insertion aid (4)

   a) comprises a curved preformation for facilitating passage through the urethra, and/or
   b) is preformed for an increased flexibility, in particular in a corrugated manner, in order to facilitate a flexible bending of the tip (7a) of the insertion aid (4), which bendability preferably reaches beyond 90 degrees, or in an ideal case beyond 135 degrees, and/or
   c) in the transition region to the distal opening (6) of the indwelling part (2) comprises a bulge-shaped, bulb-shaped or spindle-shaped cross-section expansion (7b) smoothing the transition, which, under the effect of tensile force, deforms from the proximal side in such a way that the cross-section expansion (7b) can be pulled out through the lumen of the preferably catheter-shaped indwelling part (2).

5. The system according to one of the preceding claims, **characterized in that**, in the region of a connector (10) of the urinary catheter or of the indwelling part (2), the insertion aid (4) can be secured in terms of its position by slight pressing or locking in its position, and hereto comprises a region (9) formed with a custom-fit and congruent shape.

6. The system according to one of the preceding claims, **characterized in that** the insertion aid (4)

   a) has a distal region which is made of a tubular or hose-like extension, optionally reinforcing the device (1), preferably from which a substance-accommodating reservoir unit (8) emerges in a one-piece design, and/or
   b) has a proximal region which comprises a funnel-like formation (9b), into which a structurally separate reservoir unit (8;8a) can be inserted in a tightly sealing manner via a portion (9a) that is formed with a congruent shape, especially wherein the inserted reservoir unit (8;8a) comprises on the distal end a perforable surface (9c), which can be opened by a thorn-like extension (9d) of the distal region of the insertion aid (4) in the case of a complete, terminal insertion of the reservoir (8;8a).

7. The system according to one of the preceding claims, **characterized by** a reservoir unit (8;8a) made of a material formed to be bellows-like, which, during compression of the bellows, immobilises in the respective position in an engaging manner and prevents a re-erection of the bellows, preferably wherein the reservoir unit (8;8a)

a) comprises segments (16) marked in color, by which, during the engaging compression thereof, the release of a specific volume can be coded, and/or
b) comprises segments (16), which, during the manual compression, develop respectively specific folding and/or collapsing resistances, and thus facilitate a tactilely perceptible transition from a first volume quantity to be dosed to a subsequent second volume quantity.

8. The system according to one of the preceding claims, **characterized in that** the shaft portion of the indwelling part (2) in one or more sections (13) of the shaft is preformed in a corrugated manner, by ehich the axial bending of the shaft is facilitated and elastically acting restoring forces can be reduced in the shaft.

9. The system according to one of the preceding claims, **characterized in that** the shaft portion in the urethral section or in the section between the retention balloon (3) and the proximal connector (10) of the indwelling part (2) is made of a film-like, thin-walled tube material (14), preferably comprising

a) one or more rod-like or tubular structure(s) (SE1) arranged preferably exposed in the inner lumen of the tubular film (14), in particular in order to prevent the occlusion of the drainage lumen as a result of axial twisting or kinks of the tubular film (14), and/or
b) a net-like reinforcement (SE2) permanently connected to the tubular film (14) or integrated therein, which reinforcement elastically straightens the lumen of the tubular film (14), in particular in order to hinder axial twisting and/or to return any occurred twisting back into the non-torqued starting position.

10. The system according to one of the preceding claims, **characterized in that** the distal region of the indwelling part (2) and/or of the insertion aid (4) is provided with a hood-like element (18), preferably wherein the gap space (SR) between the hood (18) and the indwelling part (2) can be filled by a particularly lubricating and/or lumen-straightening substance that is fed into the gap space (SR).

11. The system according to one of the preceding claims, **characterized in that** the hood (18) apically comprises an e.g. tap-like closure element (17), which is permanently connected to the hood (18), and which seals the outlet opening of the insertion aid (4) in a plug-like manner, and which, after disengagement thereof from the insertion aid (4), facilitates the release of the lubricating and/or lumen-straightening substance from the inner lumen of the insertion aid (4) along with the terminal, reservoirlike expansion (8;8a) thereof.

12. The system according to one of the preceding claims, **characterized in that** the unit, which consists of the indwelling part (2) and an insertion aid (4) inserted into the indwelling part (2), is inserted in a holding device (23, 25) and is reversibly fastened there, and thereby is put into a U-shaped, sling-like configuration, wherein the distal part of the unit (2, 4) is held by a device (25) opening and closing in a tongs-like or rocker-like manner, and the proximal region of the unit (2, 4) bearing the connector (10) is accommodated in the holding device (25) in preferably C-shaped recesses in such a way that a filling bellows or filling vessel (19) inserted or plugged into the connector part (10) and/or a reservoir vessel (8, 8a) possibly inserted or plugged in there can be compressed or emptied by thumb pressure.

13. The system according to claim 12, **characterized in that** the proximal region of the indwelling part (2), preferably approximately the proximal half of the indwelling part (2), in particular approximately the proximal two thirds of the indwelling part (2), is accommodated in a surrounding film-like protective pouch (27), which is connected to the holding device (23,25) that is accommodating the indwelling part (2) in a preferably permanent or even detachable manner.

14. The system according to claim 12 or 13, **characterized in that** the tongs-like or rocker-like device (25) facilitates, in the opening lateral part, an overbite-like gripping (36) of the shaft of the indwelling part (2), which, in case of a disengaging of the indwelling part (2), prevents the shaft (2) of the indwelling part (2) from being able to move out of the grip of the tongs (25) and thus being released from the holding device (25), and the overbite (36) thereof first opens in the case of a maximum opening of the rocker (26) and thereby releases the catheter shaft (2).

15. The system according to one of the preceding claims, **characterized in that** the material of the indwelling part (2) comprises a Shore hardness (x), and that the material of the insertion aid (4) comprises a Shore hardness (y), wherein the Shore hardness (y) of the material of the insertion aid (4) corresponds to or exceeds the Shore hardness (x) of the material of the indwelling part (2), in other words:

$$y = x,$$

or

$$y > x.$$

## Revendications

1. Système destiné au cathétérisme de la vessie d'un patient, comprenant un dispositif (1) et une substance, en ce que le dispositif

   a) comporte un élément de rétention (2) en forme de tige, drainant, de type cathéter qui peut être placé dans la vessie et dans l'urètre avec une lumière évacuant l'urine (2a) et au moins un orifice de drainage (6) dans la zone de l'extrémité distale (5) de l'élément de rétention (2) en forme de tige; ainsi qu'
   b) un dispositif auxiliaire d'introduction (4) rétractable, introduit dans la lumière évacuant l'urine (2a) de l'élément de rétention (2), en forme de tige, facilitant l'introduction urétrale et le passage, ledit dispositif étant de structure tubulaire ou de type tuyau flexible avec une ouverture (7) apicale pour la libération terminale de la substance par l'ouverture (7) apicale dans la zone de l'extrémité distale de l'élément de rétention (2);
   **caractérisé en ce que**
   c) la substance libérée par l'ouverture (7) apicale a des propriétés lubrifiantes et/ou redresseuses de lumière; et
   d) la partie de tige de l'élément de rétention (2) entourant la lumière évacuant l'urine (2a) s'étend d'un ballonnet de rétention (3) à un connecteur (10) proximal.

2. Système selon la revendication 1, **caractérisé en ce que** la substance peut être appliquée de manière étanche à partir de l'ouverture (7) apicale du dispositif auxiliaire d'introduction (4) à travers un orifice d'évacuation (6) dans la zone de la pointe du cathéter, de telle sorte qu'un reflux de la substance dans l'espace interstitiel (2a) entre le dispositif auxiliaire d'introduction et la lumière de cathéter de drainage est évité de manière fiable, ledit espace interstitiel étant adjacent côté proximal à l'ouverture du dispositif auxiliaire d'introduction.

3. Système selon la revendication 1 ou 2, **caractérisé en ce que** l'ouverture (7) distale du dispositif auxiliaire d'introduction (4), immédiatement adjacente à l'orifice distal de drainage (6) de l'élément de rétention (2), s'ouvre dans la lumière de drainage de l'élément de rétention (2) ou se trouve en recouvrement direct et/ou alignement commun avec l'orifice de drainage (6) de l'élément de rétention (2).

4. Système selon l'une des revendications 1 à 3, **caractérisé en ce que** la pointe (7a) du dispositif auxiliaire d'introduction (4) s'étend au-delà de l'orifice distal de drainage (6) de l'élément de rétention (2), de préférence **en ce que** la pointe (7a) du dispositif auxiliaire d'introduction (4)

   a) présente un préformage recourbé pour faciliter le passage urétral, et/ou
   b) est préformée pour une flexibilité élevée, en particulier de manière ondulée, afin de permettre une courbure flexible de la pointe (7a) du dispositif auxiliaire d'introduction (4), la flexibilité étant de préférence supérieure à 90 degrés, ou dans le cas idéal supérieure à 135 degrés, et/ou
   c) comporte dans la zone de transition vers l'orifice distal (6) de l'élément de rétention (2) une extension de section transversale (7b) lissante la transition, en forme de bourrelet ou de bulbe ou de broche qui, sous l'effet d'une traction exercée depuis le côté proximal, se déforme de telle sorte que l'extension de section transversale (7b) peut être retirée par la lumière de l'élément de rétention (2) de préférence en forme de cathéter.

5. Système selon l'une des revendications précédentes, **caractérisé en ce que** le dispositif auxiliaire d'introduction (4) peut être bloqué dans sa position dans la zone d'un connecteur (10) du cathéter urinaire ou de l'élément de rétention (2) par une légère pression ou encliquetage, et présente à cet effet une zone (9) formée de manière précise, de forme congruente.

6. Système selon l'une des revendications précédentes, **caractérisé en ce que** le dispositif auxiliaire d'introduction (4)

   a) présente une zone distale, qui consiste en un prolongement tubulaire ou de type tuyau flexible, renforçant éventuellement le dispositif (1), d'où résulte de préférence directement, sous forme d'une construction monobloc, une unité de réservoir (8) de réception de substance, et/ou

b) comporte une zone proximale, qui présente une forme de type entonnoir (9b), dans laquelle une unité de réservoir (8; 8a) de structure séparée peut être insérée de manière étanche par l'intermédiaire de la partie (9a) formée de forme congruente, en particulier **en ce que** l'unité de réservoir (8; 8a) insérée présente au niveau de l'extrémité distale une surface (9c) qui peut être perforée, laquelle peut être ouverte par un prolongement (9d) en forme de broche de la zone distale du dispositif auxiliaire d'introduction (4) à introduction complète et terminale du réservoir (8; 8a).

7. Système selon l'une des revendications précédentes, **caractérisé par** une unité de réservoir (8; 8a) constituée d'un matériau formé de type soufflet, qui se fixe à compression du soufflet de préférence dans la position respective de manière verrouillable et empêche un redressement du soufflet, de préférence en ce que l'unité de réservoir (8; 8a)

a) comporte des segments (16) marqués en couleur, ce qui permet decoder la sortie d'un volume défini lors de leur compression par encliquetage, et/ou

b) comporte des segments (16) qui, lors de la compression manuelle, développent chacun des résistances spécifiques au pliage et/ou à l'affaissement, et permettent ainsi une transition tactilement perceptible d'un premier débit volumique à doser à un deuxième débit volumique suivant.

8. Système selon l'une des revendications précédentes, **caractérisé en ce que** la partie de tige de l'élément de rétention (2) est préformée de manière ondulée dans une ou plusieurs sections (13) de la tige, ce qui facilite la flexion axiale de la tige et permet de réduire des forces de rappel agissant élastiquement dans la tige.

9. Système selon l'une des revendications précédentes, **caractérisé en ce que** la partie de tige dans la section urétrale ou dans la section entre le ballonnet de rétention (3) et le connecteur (10) proximal de l'élément de rétention (2) est constituée d'un matériau tubulaire à parois minces de type film (14), comprenant de préférence

a) une ou plusieurs structure(s) (SE1) en forme de tige ou de tube, de préférence disposée(s) librement dans la lumière intérieure du film tubulaire (14), en particulier pour éviter l'obturation de la lumière de drainage suite à des torsions axiales ou flambages du film tubulaire (14) et/ou

b) une armature réticulée (SE2) reliée à demeure au film tubulaire (14) ou intégrée dans celui-ci, ladite armature redressant élastiquement la lumière du film tubulaire (14), en particulier pour rendre plus difficile des torsions axiales et/ou ramener des torsions qui se sont pro- duites dans la situation initiale non tordue.

10. Système selon l'une des revendications précédentes, **caractérisé en ce que** la zone distale de l'élément de rétention (2) et/ou du dispositif auxiliaire d'introduction (4) est pourvu d'un élément en forme de capuchon (18), de préférence **en ce que** l'espace interstitiel (SR) entre le capuchon (18) et l'élément de rétention (2) peut être rempli par une substance, en particulier lubrifiante et/ou redressant la lumière, introduite dans l'espace interstitiel (SR).

11. Système selon l'une des revendications précédentes, **caractérisé en ce que** le capuchon (18) comporte au niveau apical un élément de fermeture (17), par exemple en forme de pivot, relié de manière solidaire au capuchon (18), élément de fermeture qui obture à la manière d'un bouchon l'orifice de sortie du dispositif auxiliaire d'introduction (4), et lequel après avoir été détaché du dispositif auxiliaire d'introduction (4) permet la libération de la substance lubrifiante et/ou redressant la lumière de la lumière intérieure du dispositif auxiliaire d'introduction (4) ainsi que son extension terminale en forme de réservoir (8; 8a).

12. Système selon l'une des revendications précédentes, **caractérisé en ce que** l'unité comprenant l'élément de rétention (2) et un dispositif auxiliaire d'introduction (4) enfiché dans l'élément de rétention (2) est insérée dans un dispositif de retenue (23, 25) et y est fixée de manière réversible, et prend ainsi une configuration en forme de boucle en U, **en ce que** la partie distale de l'unité (2, 4) est maintenue par un dispositif (25) de type pince ou bascule s'ouvrant et se fermant, et la zone proximale portant le connecteur (10) de l'unité (2, 4) dans le dispositif de retenue (25) est logée dans des encoches de préférence en forme de C, de telle sorte qu'un soufflet de remplissage ou récipient de remplissage (19) fixé ou inséré à ou dans la partie de connecteur (10) et/ou un récipient de réservoir (8; 8a) qui y est le cas échéant fixé ou inséré peut être comprimé et/ou vidé par pression du pouce.

13. Système selon la revendication 12, **caractérisé en ce que** la zone proximale de l'élément de rétention (2), de préférence approximativement la moitié proximale de l'élément de rétention (2), en particulier approximativement les deux tiers proximaux de l'élément de rétention (2), est logée dans une poche de protection (27) enveloppante de type film, qui est reliée de préférence de manière fixe ou également amovible au dispositif de retenue (23, 25) logeant l'élément de rétention (2).

**14.** Système selon l'une des revendications 12 ou 13, **caractérisé en ce que** le dispositif (25) de type pince ou bascule dans la partie latérale ouvrante permet une préhension (36) en surplomb de la tige de l'élément de rétention (2), ce qui empêche la tige (2) de l'élément de rétention (2) de se dégager de la prise de la pince (25) lors du desserrage de l'élément de rétention (2) et d'être ainsi libérée du dispositif de retenue (25), et dont la mâchoire supérieure (36) ne s'ouvre qu'à ouverture maximale de la bascule (26), libérant ainsi la tige du cathéter (2).

**15.** Système selon l'une des revendications précédentes, **caractérisé en ce que** le matériau de l'élément de rétention (2) présente une dureté Shore (x), et que le matériau du dispositif auxiliaire d'introduction (4) présente une dureté Shore (y), **en ce que** la dureté Shore (y) du matériau du dispositif auxiliaire d'introduction (4) correspond à la dureté Shore (x) du matériau de l'élément de rétention (2) ou lui est supérieure, soit:

$$y = x,$$

ou

$$y > x.$$

Abb.1

Abb.2

Abb.2a

Abb.2b

Abb.2c

Abb.2d

Abb.3a

Abb.3b

Abb.4

Abb.5

Abb.6

Abb.7

Abb.7a

23

Abb.8

28b

34    33    28

35    32

29    31

Z

Z    28a

Z    30    29

**EP 4 025 286 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1448259 A
- WO 2015002282 A

- EP 0935977 A2